# EUROPÄISCHE PATENTSCHRIFT

(11) **EP 3 661 605 B1**
(45) Veröffentlichungstag und Bekanntmachung des Hinweises auf die Patenterteilung: **25.05.2022**
(21) Anmeldenummer: 18742421.3
(22) Anmeldetag: 05.07.2018
(51) Int. Cl.: A61Q 19/10, A61K 8/34, A61K 8/365, A61K 8/37, A61K 8/73, A61K 8/92, A61K 8/19, A61K 8/04, A61K 8/02, A61K 8/36

(54) **KOSMETIKUM ZUR GESICHTSREINIGUNG**
COSMETIC FOR FACIAL CLEANSING
PRODUIT COSMÉTIQUE POUR LE NETTOYAGE DU VISAGE

(30) Priorität: 02.08.2017 DE 102017213331
(43) Veröffentlichungstag der Anmeldung: 10.06.2020
(73) Patentinhaber: Beiersdorf AG, 20253 Hamburg (DE)
(72) Erfinder: VON DAVIER, Anabelle, 22391 Hamburg (DE); WERNER, Regine, 29553 Bienenbüttel (DE)
(74) Vertreter: Beiersdorf AG
(86) Internationale Anmeldenummer: PCT/EP2018/068229
(87) Internationale Veröffentlichungsnummer: WO 2019/025115

(56) Entgegenhaltungen:
- EP-A1- 2 886 625
- EP-A2- 0 745 665
- EP-A2- 2 620 138
- WO-A1-2008/041045
- DE-A1- 10 327 432
- DE-A1- 19 849 218
- DE-A1-102015 219 066
- DATABASE WPI Week 200116 Thomson Scientific, London, GB; AN 2001-151072 XP002785117, "Foaming cosmetics comprises at least two separate compositions of a cosmetic composition containing carbonic acid salt and a cosmetic composition containing an acidic ingredient", & JP 2000 297007 A (POLA CHEM IND INC) 24. Oktober 2000 (2000-10-24)
- DATABASE GNPD [Online] MINTEL; 31. Mai 2017 (2017-05-31), anonymous: "Foam (Pure Milk)", XP055510275, gefunden im www.gnpd.com Database accession no. 4856463

## Beschreibung

Die vorliegende Erfindung betrifft ein kosmetisches Produkt bestehend aus
a) einem Packmittel mit zwei räumlich voneinander getrennten Vorratskammern deren Inhalt aus einer gemeinsamen Öffnung entnommen werden kann,
b) eine sich in der ersten Vorratskammer befindliche erste Zubereitung enthaltend eine wässrige, saure Zubereitung mit einer Säure, Wasser und einen Gelbildner,
c) eine sich in der zweiten Vorratskammer befindliche zweite Zubereitung enthaltend einen oder mehrere Emulgatoren, ein oder mehrere Carbonate sowie ein oder mehrere lipophile Verbindungen.

Die Produktion von kosmetischen Reinigungsmitteln zeigt seit Jahren eine steigende Tendenz. Dies ist vor allem auf das zunehmende Gesundheitsbewußtsein und Hygienebedürfnis der Verbraucher zurückzuführen.

Reinigung des menschlichen Körpers bedeutet das Entfernen von (Umwelt-) Schmutz und bewirkt damit eine Erhöhung des psychischen und physischen Wohlbefindens. Die Reinigung der Oberfläche von Haut und Haaren ist ein sehr komplexer, von vielen Parametern abhängiger Vorgang. Zum einen sollen von außen kommende Substanzen wie beispielsweise Kohlenwasserstoffe oder anorganische Pigmente aus unterschiedlichsten Umfeldern sowie Rückstände von Kosmetika oder auch unerwünschte Mikroorganismen möglichst vollständig entfernt werden. Zum anderen sind körpereigene Ausscheidungen wie Schweiß, Sebum, Haut- und Haarschuppen ohne tiefgreifende Eingriffe in das physiologische Gleichgewicht abzuwaschen.

Kosmetische Reinigungspräparate sind sogenannte "rinse off" Präparate, welche nach der Anwendung von der Haut abgespült werden. Sie werden in aller Regel in Form eines Schaums mit Wasser auf die zu reinigenden Körperpartien aufgetragen. Basis aller kosmetischen oder dermatologischen Reinigungspräparate sind daher waschaktive Tenside mit einem HLB-Wert von größer/gleich 13. Tenside sind amphiphile Stoffe, die organische, unpolare Substanzen in Wasser lösen können. Sie zeichnen sich durch ein ambivalentes Verhalten gegenüber Wasser und Lipiden aus: Das Tensidmolekül enthält mindestens je eine hydrophile und eine lipophile Gruppe, die die Anlagerung an der Grenzfläche zwischen diesen beiden Substanzklassen ermöglichen. Auf diese Weise sorgen Tenside für eine Herabsetzung der Oberflächenspannung des Wassers, die Benetzung der Haut, die Erleichterung der Schmutzentfernung und -lösung, ein leichtes Abspülen und - je nach Wunsch - auch für Schaumregulierung. Damit ist die Grundlage für die Schmutzentfernung lipidhaltiger Verschmutzungen gegeben.

Eine besondere Form von Reinigungszubereitungen stellen die so genannten Gesichtsmasken dar. Bei dieser speziellen Form der Gesichtsreinigung wird eine relativ hoch viskose Paste in konzentrierter Form auf die Haut aufgebracht die man anschließend über einen längeren Zeitraum als bei normalen Reinigungszubereitungen üblich, auf die Haut einwirken lässt. Diese, häufig auch einem Peeling-Effekt auslösenden Zubereitungen müssen besonders hautfreundlich sein.

Nachteilig am Stande der Technik ist der Umstand, dass, wenn es sich um schäumende Gesichtsmasken handeln soll, die Zubereitungen mit Tensiden versetzt werden müssen, welche den (zeitlich) stabilen Schaum erzeugen. Im Vergleich zu nicht schäumenden Emulsionen sind diese Zubereitungen dann weniger hautfreundlich und können bei empfindlichen Menschen zu Hautreizungen oder zum übermäßigen Austrocknen der Haut führen.

Es war daher die Aufgabe der vorliegenden Erfindung, eine besonders hautfreundliche, gut schäumbare Gesichtsmaske mit stabilen Schaum zu entwickeln.

Viele Gesichtsreinigungsmasken enthalten als Peeling-Material Kreide (Calciumcarbonat). Nachteilig am Stande der Technik ist jedoch der Umstand, dass sich Kreide, wenn sie in größeren Mengen in eine Emulsion eingearbeitet werden soll, dazu führt, dass diese Emulsion dünnflüssig und instabil wird.

Es war daher die Aufgabe der vorliegenden Erfindung, eine Gesichtsreinigungsmaske auf der Basis einer Emulsion zu entwickeln, die einerseits gut schäumt und in die sich andererseits größere Mengen an Kreide stabil einarbeiten lassen.

Nicht zuletzt wurden in den letzten Jahrzehnten häufig farbige Kunststoffpartikel (beispielsweise aus HDPE) in Gesichtsmasken eingearbeitet, die zur Erhöhung der optischen Attraktivität beitragen und als Peeling-Material dienen sollten.

Nachteilig an diesem Stande der Technik ist jedoch der Umstand, dass von derartigen "Mikroplastik"-Partikeln aufgrund ihrer Langlebigkeit eine nicht akzeptable Belastung für die Umwelt ausgeht.

Es war daher die Aufgabe der vorliegenden Erfindung, die optische Attraktivität von Gesichtsreinigungsmasken auf umweltfreundliche Art zu erhöhen.

Überraschend gelöst werden diese Aufgaben durch ein kosmetisches Produkt nach Anspruch 1.

Zwar kennt der Stand der Technik die Offenbarungen der DE 103 27 432 A1, DE198 49 218 A1, EP 2 886 625 A1, WO 2008/041045 A1, EP 0 745 665 A2, EP 2 620 138 A2, DE 10 2015 219 006 A1, JP 19990103545 (Anmeldenummer:2001-151072) sowie den Datenbank-Eintrag in der GNPD Datenbank "Mintel" Eintragungsnummer 4856463, doch konnten diese Veröffentlichungen nicht den Weg zur vorliegenden Erfindung weisen.

Dabei ist es erfindungsgemäß vorteilhaft, wenn als Packmittel eine Tube aus LDPE, HDPE, PP oder Abmischungen eingesetzt wird.

Dabei ist es erfindungsgemäß bevorzugt, wenn als Packmittel eine Tube aus LDPE eingesetzt wird.

Es ist erfindungsgemäß von Vorteil, wenn die gemeinsame Öffnung des Packmittels kleeblattförmig ist. In diesem Falle lassen sich, insbesondere bei unterschiedlich eingefärbten Einzelzubereitungen attraktive optische Effekte erzielen.

Darüber hinaus sind erfindungsgemäß vorteilhafte Ausführungsformen der vorliegenden Erfindung dadurch gekennzeichnet, dass die Inhalte beider Vorratskammern gleichzeitig im Volumen-Verhältnis (erste Kammer zu zweite Kammer) 1,2:1 bis 1:1,2 entnommen werden. Erfindungsgemäß bevorzugt ist dabei ein Volumen-Verhältnis (erste Kammer zu zweite Kammer) 1:1 bis 1:1.

Es ist erfindungsgemäß besonders bevorzugt, wenn die Zubereitungen in den beiden Vorratskammern frei sind von oberflächenaktiven Stoffen mit einem HLB-Wert von größer/gleich 13. Dabei können die HLB-Werte den üblichen Tabellenwerken entnommen werden (z.B. H:P: Fiedler, Lexikon der Hilfsstoffe in Pharmazie, Kosmetik und angrenzenden Gebieten). Diese Zubereitungen gelten dann als "tensidfrei".

Es ist erfindungsgemäß vorteilhaft, wenn die Zubereitung in der ersten Kammer einen pH-Wert von 2 bis 4,5 aufweist.

In einem solchen Falle ist es erfindungsgemäß vorteilhaft, wenn die Zubereitung in der ersten Kammer Glycolsäure in einer Menge von 5 bis 15 Gewichts-%, bezogen auf das Gesamtgewicht der ersten Zubereitung beträgt. Die erfindungsgemäß bevorzugte Einsatzkonzentration beträgt von 8 bis 12 Gewichts-%, bezogen auf das Gesamtgewicht der ersten Zubereitung.

Vorteilhaft im Sinne der vorliegenden Erfindung ist es, wenn die Zubereitung in der ersten Kammer als Gelbildner Hydroxyethylcellulose und/oder Xanthangummi enthält. Dabei ist es erfindungsgemäß bevorzugt, wenn diese Zubereitung Hydroxyethylcellulose und Xanthangummi enthält.

Es ist erfindungsgemäß vorteilhaft, wenn die Zubereitung in der ersten Kammer Hydroxyethylcellulose in einer Konzentration von 0,1 bis 1 Gewichts-%, bezogen auf das Gesamtgewicht der ersten Zubereitung, enthält.

Es ist erfindungsgemäß vorteilhaft, wenn die Zubereitung in der ersten Kammer Xanthangummi in einer Konzentration von 0,1 bis 1,5 Gewichts-%, bezogen auf das Gesamtgewicht der ersten Zubereitung, enthält.

Erfindungsgemäß vorteilhaft beträgt die Viskosität der Zubereitung in der ersten Kammer von 4000 bis 20.000 mPas.

Die im Rahmen der vorliegenden Schrift aufgeführten Viskositätswerte der Zubereitungen werden dabei mit Hilfe eines Rotationsrheometer ( Rheomat 123) mit VT02-Messsystemen der Gesellschaft proRheo ermittelt (Temperatur: 25°C, Messkörper 1 und 2, Rotorgeschwindigkeit 62.5 1/min).

Erfindungsgemäß vorteilhaft enthält die Zubereitung in der ersten Kammer Wasser in einer Konzentration von 65 bis 85 Gewichts-%, bezogen auf das Gesamtgewicht der ersten Zubereitung.

Erfindungsgemäß vorteilhafte Ausführungsformen der vorliegenden Erfindung sind dadurch gekennzeichnet, dass die Zubereitung in der ersten Kammer Farbpigmente enthält. Diese können in einer Konzentration von 0,0001 bis 1 Gewichts-%, bezogen auf das Gesamtgewicht der ersten Zubereitung in dieser eingesetzt werden.

Als erfindungsgemäß vorteilhafte Farbstoffe kommen dabei in Frage: Cl 14720, Cl 61565, Cl 60725, Cl 74160, Cl 61570, Cl 42090, Cl 47005, Cl 17200, Cl 16035, Cl 28440, Cl 15985, Cl 42053, Cl 10316.

Die Zusammensetzung der Zubereitung in der zweiten Kammer wird durch die folgenden Parameter und Inhaltsstoffe charakterisiert:
So ist es erfindungsgemäß bevorzugt, wenn die Zubereitung in der zweiten Kammer als Emulgator Glycerylstearat (selbstemulgierend, INCI: Glyceryl Stearate SE) enthält.

In einem solchen Falle ist es erfindungsgemäß vorteilhaft, wenn die Zubereitung in der zweiten Kammer Glycerylstearat (selbstemulgierend) in einer Menge von 1 bis 8 Gewichts-%, bezogen auf das Gesamtgewicht der zweiten Zubereitung, enthält, wobei die erfindungsgemäß bevorzugte Einsatzkonzentration von 4 bis 6 Gewichts-%, bezogen auf das Gesamtgewicht der zweiten Zubereitung beträgt.

Erfindungsgemäß vorteilhafte Ausführungsformen der vorliegenden Erfindung sind dadurch gekennzeichnet, dass die Zubereitung in der ersten Kammer Glycerin enthält. Dabei wird Glycerin erfindungsgemäß bevorzugt in einer Einsatzkonzentration von 5 bis 15 Gewichts-%, bezogen auf das Gesamtgewicht der ersten Zubereitung, eingesetzt.

Es ist erfindungsgemäß vorteilhaft, wenn die Zubereitung in der zweiten Kammer Calciumcarbonat in einer Menge von 5 bis 15 Gewichts-%, bezogen auf das Gesamtgewicht der zweiten Zubereitung, enthält, wobei ein Konzentrationsbereich 8 von bis 12 Gewichts-%, bezogen auf das Gesamtgewicht der zweiten Zubereitung, besonders bevorzugt ist.

Das erfindungsgemäße Calciumcarbonat wird erfindungsgemäß vorteilhaft als Feststoff der Zubereitung in der zweiten Kammer zugesetzt. Dabei ist es erfindungsgemäß von besonderem Vorteil, wenn Calciumcarbonat mit einer durchschnittlichen Partikelgröße von 1 bis 3µm, eingesetzt wird, um beim Vermischen mit der Zubereitung der ersten Kammer einerseits ausreichend Treibgas zum Aufschäumen der Zubereitung zu erzeugen.

Es ist erfindungsgemäß vorteilhaft, wenn die Zubereitung in der zweiten Kammer Sheabutter enthält. Erfindungsgemäß bevorzugt ist dabei eine Einsatzkonzentration von 10 bis 30 Gewichts-%, bezogen auf das Gesamtgewicht der zweiten Zubereitung.

Neben Sheabutter kann die Zubereitung in der zweiten Kammer auch noch weitere lipophile Verbindungen enthalten.

Es ist vorteilhaft im Sinne der vorliegenden Erfindung, wenn die Zubereitung in der zweiten Kammer Cetearylalkohol und/oder Xanthangummi enthält. Dabei ist ein Gehalt an Cetearylalkohol und Xanthangummi erfindungsgemäß bevorzugt.

Die erfindungsgemäß vorteilhafte Einsatzkonzentration von Cetearylalkohol beträgt von1 bis 3 Gewichts-%, bezogen auf das Gesamtgewicht der zweiten Zubereitung.

Die erfindungsgemäß vorteilhafte Einsatzkonzentration von Xanthangummi beträgt von 1 bis 3 Gewichts-%, bezogen auf das Gesamtgewicht der zweiten Zubereitung.

Erfindungsgemäß vorteilhaft beträgt die Viskosität der Zubereitung in der zweiten Kammer von 4000 bis 20000 mPas.

Die erfindungsgemäßen Zubereitungen in der ersten und zweiten Kammer können darüber hinaus noch weitere, für Gesichtsmasken übliche Inhaltsstoffe in den üblichen Einsatzkonzentrationen enthalten.

Es ist dabei erfindungsgemäß von Vorteil, wenn die erfindungsgemäßen Zubereitungen frei sind von Polyethylenglycol, Polyethylenglycolethern und Polyethylenglycolestern (sogenannten PEG-Derivaten).

Erfindungsgemäß vorteilhafte Ausführungsformen der vorliegenden Erfindung sind dadurch gekennzeichnet, dass mindestens eine der Zubereitungen Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthalten. Diese befinden sich erfindungsgemäß bevorzugt in der ersten Zubereitung, die auch vorteilhaft Wasser enthält.

Diese Verbindungen können einzeln oder in Kombination in den erfindungsgemäßen Zubereitungen vorliegen. Die erfindungsgemäße Einsatzkonzentration (Einzelkonzentration) beträgt für diese Inhaltsstoffe von 0,01 bis 5 Gewichts-%, bezogen auf das Gesamtgewicht der Zubereitung.

Es ist insbesondere der Einsatz von Ethylhexylglycerin erfindungsgemäß besonders vorteilhaft. Die erfindungsgemäß vorteilhafte Einsatzkonzentration für Ethylhexylglycerin beträgt von 0.1 bis 1 Gewichts-%, bezogen auf das Gesamtgewicht der Zubereitung in der einzelnen Kammer.

Es ist erfindungsgemäß vorteilhaft, wenn die erfindungsgemäßen Zubereitungen Phenoxyethanol, 4-Hydroxyacetophenon und/oder Ethanol enthalten.

Phenoxyethanol kann dabei erfindungsgemäß vorteilhaft in einer Konzentration von 0,1 bis 0,9 Gewichts-%, bezogen auf das Gesamtgewicht der Zubereitung in der einzelnen Kammer eingesetzt werden.

Erfindungsgemäß bevorzugt ist der Einsatz einer Kombination aus Phenoxyethanol und Ethylhexylglycerin.

Erfindungsgemäß bevorzugte Ausführungsformen werden dadurch erhalten, dass die Zubereitungen keine Parabene (insbesondere Propyl- und Butylparaben), sowie kein 3-lod-2-propinylbutylcarbamat, Methylisothiazolinon, Chlormethylisothiazolinon und DMDM-Hydantoin enthält, also frei ist von diesen Inhaltsstoffen.

Es ist erfindungsgemäß von Vorteil, wenn die erfindungsgemäßen Zubereitungen einen oder mehrere der Parfümstoffe gewählt aus der Liste der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Hydroxyisohexyl 3-Cyclohexencarboxaldehyd, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin, enthalten.

Erfindungsgemäß vorteilhafte Ausführungsformen der vorliegenden Erfindung sind auch dadurch gekennzeichnet, dass die Zubereitungen eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Glycyrrhetinsäure, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthalten.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**Beispielrezeptur:**

| | **äußere Kammer** | **innere Kammer** |
|---|---|---|
| Butyrospermum Parkii Butter | 0 | 15 |
| Glyceryl Stearate SE | 0 | 5 |
| Calcium Carbonate | 0 | 10 |
| Parfum | 0,1 | 0 |
| Glycerin + Aqua | 10 | 0 |
| Aqua + Sodium Hydroxide | 3,074 | 0 |
| Glycolic Acid + Aqua | 10 | 0 |
| Hydroxyethylcellulose | 1 | 0 |
| Xanthan Gum | 1 | 0 |
| Cetearyl Alcohol | 0 | 0,5 |
| Xanthan Gum | 0 | 0,5 |
| Cl 10316 | 0,0006 | 0 |
| Aqua | ad 100 | Ad 100 |

**Packmittel:** LDPE-Tube mit 2 Kammern (äußere und innere Kammer.

## Patentansprüche

1. Kosmetisches Produkt bestehend aus
a) einem Packmittel mit zwei räumlich voneinander getrennten Vorratskammern deren Inhalt aus einer gemeinsamen Öffnung entnommen werden kann,
b) eine sich in der ersten Vorratskammer befindliche erste Zubereitung enthaltend eine wässrige, saure Zubereitung mit einer Säure, Wasser und einem Gelbildner, wobei als Säure Glycolsäure enthalten ist,
c) eine sich in der zweiten Vorratskammer befindliche zweite Zubereitung enthaltend einen oder mehrere Emulgatoren, ein oder mehrere Carbonate sowie ein oder mehrere lipophile Verbindungen, **dadurch gekennzeichnet, dass** die Zubereitung in der zweiten Kammer als Carbonat Calciumcarbonat enthält.

2. Kosmetisches Produkt nach Anspruch 1, **dadurch gekennzeichnet, dass** als Packmittel eine Tube aus LDPE eingesetzt wird.

3. Kosmetisches Produkt nach einem der vorhergehenden Ansprüche, **dadurch gekennzeichnet, dass** die gemeinsame Öffnung des Packmittels kleeblattförmig ist.

4. Kosmetisches Produkt nach einem der vorhergehenden Ansprüche, **dadurch gekennzeichnet, dass** die Inhalte beider Vorratskammern gleichzeitig im Volumen-Verhältnis (erste Kammer zu zweite Kammer) 1,2:1 bis 1:1,2 entnommen werden.

5. Kosmetisches Produkt nach einem der vorhergehenden Ansprüche, **dadurch gekennzeichnet**, das die Zubereitungen in den beiden Vorratskammern frei sind von oberflächenaktiven Stoffen mit einem HLB-Wert von größer/gleich 13.

6. Kosmetisches Produkt nach einem der vorhergehenden Ansprüche **dadurch gekennzeichnet, dass** die Zubereitung in der ersten Kammer einen pH-Wert von 2 bis 4,5 aufweist.

7. Kosmetisches Produkt nach einem der vorhergehenden Ansprüche, **dadurch gekennzeichnet, dass** die Zubereitung in der ersten Kammer Glycolsäure in einer Menge von 5 bis15 Gewichts-%, bezogen auf das Gesamtgewicht der ersten Zubereitung beträgt.

8. Kosmetisches Produkt nach einem der vorhergehenden Ansprüche, **dadurch gekennzeichnet, dass** die Zubereitung in der ersten Kammer als Gelbildner Hydroxyethylcellulose und/oder Xanthangummi enthält.

9. Kosmetisches Produkt nach einem der vorhergehenden Ansprüche, **dadurch gekennzeichnet, dass** die Zubereitung in der ersten Kammer Farbpigmente enthält.

10. Kosmetisches Produkt nach einem der vorhergehenden Ansprüche, **dadurch gekennzeichnet, dass** die Zubereitung in der zweiten Kammer als Emulgator Glycerylstearat (selbstemulgierend, INCI: Glyceryl Stearate SE) enthält.

11. Kosmetisches Produkt nach einem der vorhergehenden Ansprüche, **dadurch gekennzeichnet, dass** die Zubereitung in der zweiten Kammer Glycerylstearat (selbstemulgierend) in einer Menge von 1 bis 8 Gewichts-%, bezogen auf das Gesamtgewicht der zweiten Zubereitung, enthält.

12. Kosmetisches Produkt nach einem der vorhergehenden Ansprüche, **dadurch gekennzeichnet, dass** die Zubereitung in der zweiten Kammer Glycerin enthält.

13. Kosmetisches Produkt nach einem der vorhergehenden Ansprüche, **dadurch gekennzeichnet, dass** die Zubereitung in der zweiten Kammer Calciumcarbonat in einer Menge von 8 bis 12 Gewichts-%, bezogen auf das Gesamtgewicht der zweiten Zubereitung, enthält.

14. Kosmetisches Produkt nach einem der vorhergehenden Ansprüche, **dadurch gekennzeichnet, dass** die Zubereitung in der zweiten Kammer Sheabutter enthält.

15. Kosmetisches Produkt nach einem der vorhergehenden Ansprüche, **dadurch gekennzeichnet, dass** die Zubereitung in der zweiten Kammer Cetearylalkohol und/oder Xanthangummi enthält.

## Claims

1. Cosmetic product consisting of
a) a packaging having two stock chambers spatially separated from each other, the contents of which may be withdrawn from a common opening,
b) a first preparation in the first stock chamber comprising an aqueous, acidic preparation with an acid, water and a gel former, wherein glycolic acid is present as acid,
c) a second preparation in the second stock chamber comprising one or more emulsifiers, one or more carbonates and also one or more lipophilic compounds, **characterized in that** the preparation in the second chamber comprises calcium carbonate as carbonate.

2. Cosmetic product according to Claim 1, **characterized in that** the packaging used is a tube composed of LDPE.

3. Cosmetic product according to either of the preceding claims, **characterized in that** the common opening of the packaging is cloverleaf-shaped.

4. Cosmetic product according to any of the preceding claims, **characterized in that** the contents of both stock chambers are removed simultaneously at a ratio by volume (first chamber to second chamber) of from 1.2:1 to 1:1.2.

5. Cosmetic product according to any of the preceding claims, **characterized in that** the preparations in both stock chambers are free from surface-active substances having an HLB value of greater than/equal to 13.

6. Cosmetic product according to any of the preceding claims, **characterized in that** the preparation in the first chamber has a pH of 2 to 4.5.

7. Cosmetic product according to any of the preceding claims, **characterized in that** the preparation in the first chamber is glycolic acid in an amount of 5 to 15% by weight, based on the total weight of the first preparation.

8. Cosmetic product according to any of the preceding claims, **characterized in that** the preparation in the first chamber comprises hydroxyethyl cellulose and/or xanthan gum as gel former.

9. Cosmetic product according to any of the preceding claims, **characterized in that** the preparation in the first chamber comprises colour pigments.

10. Cosmetic product according to any of the preceding claims, **characterized in that** the preparation in the second chamber comprises glyceryl stearate (self-emulsifying, INCI: Glyceryl Stearate SE) as emulsifier.

11. Cosmetic product according to any of the preceding claims, **characterized in that** the preparation in the second chamber comprises glyceryl stearate (self-emulsifying) in an amount of 1 to 8% by weight, based on the total weight of the second preparation.

12. Cosmetic product according to any of the preceding claims, **characterized in that** the preparation in the second chamber comprises glycerin.

13. Cosmetic product according to any of the preceding claims, **characterized in that** the preparation in the second chamber comprises calcium carbonate in an amount of 8 to 12% by weight, based on the total weight of the second preparation.

14. Cosmetic product according to any of the preceding claims, **characterized in that** the preparation in the second chamber comprises shea butter.

15. Cosmetic product according to any of the preceding claims, **characterized in that** the preparation in the second chamber comprises cetearyl alcohol and/or xanthan gum.

## Revendications

1. Produit cosmétique constitué
a) d'un moyen d'emballage comportant deux compartiments de réserve séparés l'un de l'autre dans l'espace, dont le contenu peut être prélevé à partir d'une ouverture commune,
b) d'une première préparation se trouvant dans le premier compartiment de réserve contenant une préparation aqueuse acide comportant un acide, de l'eau et un agent gélifiant, de l'acide glycolique étant contenu en tant qu'acide,
c) d'une deuxième préparation se trouvant dans le deuxième compartiment de réserve contenant un ou plusieurs émulsifiants, un ou plusieurs carbonates ainsi qu'un ou plusieurs composés lipophiles, **caractérisé en ce que** la préparation dans le deuxième compartiment contient du carbonate de calcium en tant que carbonate.

2. Produit cosmétique selon la revendication 1, **caractérisé en ce qu'**un tube de LDPE est utilisé en tant que moyen d'emballage.

3. Produit cosmétique selon l'une quelconque des revendications précédentes, **caractérisé en ce que** l'ouverture commune du moyen d'emballage est en forme de feuille de trèfle.

4. Produit cosmétique selon l'une quelconque des revendications précédentes, **caractérisé en ce que** les contenus des deux compartiments de réserve sont prélevés simultanément en un rapport en volume (premier compartiment sur deuxième compartiment) de 1,2:1 à 1:1,2.

5. Produit cosmétique selon l'une quelconque des revendications précédentes, **caractérisé en ce que** les préparations dans les deux compartiments de réserve sont exemptes de substances actives en surface dotées d'une valeur de HLB supérieure/égale à 13.

6. Produit cosmétique selon l'une quelconque des revendications précédentes, **caractérisé en ce que** la préparation dans le premier compartiment présente une valeur de pH de 2 à 4,5.

7. Produit cosmétique selon l'une quelconque des revendications précédentes, **caractérisé en ce que** la préparation dans le premier compartiment est de l'acide glycolique en une quantité de 5 à 15 % en poids, par rapport au poids total de la première préparation.

8. Produit cosmétique selon l'une quelconque des revendications précédentes, **caractérisé en ce que** la préparation dans le premier compartiment contient hydroxyéthylcellulose et/ou une gomme xanthane en tant qu'agent gélifiant.

9. Produit cosmétique selon l'une quelconque des revendications précédentes, **caractérisé en ce que** la préparation dans le premier compartiment contient des pigments colorés.

10. Produit cosmétique selon l'une quelconque des revendications précédentes, **caractérisé en ce que** la préparation dans le deuxième compartiment contient du stéarate de glycéryle en tant qu'émulsifiant (auto-émulsifiant, INCI : Glyceryl Stearate SE).

11. Produit cosmétique selon l'une quelconque des revendications précédentes, **caractérisé en ce que** la préparation dans le deuxième compartiment contient du stéarate de glycéryle (auto-émulsifiant) en une quantité de 1 à 8 % en poids, par rapport au poids total de la deuxième préparation.

12. Produit cosmétique selon l'une quelconque des revendications précédentes, **caractérisé en ce que** la préparation dans le deuxième compartiment contient de la glycérine.

13. Produit cosmétique selon l'une quelconque des revendications précédentes, **caractérisé en ce que** la préparation dans le deuxième compartiment contient du carbonate de calcium en une quantité de 8 à 12 % en poids, par rapport au poids total de la deuxième préparation.

14. Produit cosmétique selon l'une quelconque des revendications précédentes, **caractérisé en ce que** la préparation dans le deuxième compartiment contient du beurre de karité.

15. Produit cosmétique selon l'une quelconque des revendications précédentes, **caractérisé en ce que** la préparation dans le deuxième compartiment contient de l'alcool cétéarylique et/ou une gomme xanthane.
